# EUROPEAN PATENT APPLICATION

(11) **EP 4 443 129 A1**
(43) Date of publication of application: **09.10.2024**
(21) Application number: 24153171.4
(22) Date of filing: 22.01.2024
(51) Int. Cl.: G01N 3/40, G01N 35/00, G01N 33/00, G01N 33/15

(54) **SYSTEM AND METHOD FOR MEASURING HARDNESS OF MOLDED PRODUCT**

(30) Priority: 03.04.2023 JP 2023060283
(71) Applicant: Kikusui Seisakusho Ltd., Nakagyo-ku Kyoto-shi Kyoto 604-8483 (JP)
(72) Inventor: HIROSHI, SUZUKI, Kyoto-shi, 604-8483 (JP)
(74) Representative: TBK

(57) **Abstract**

The invention provides a hardness measurement system for a molded product P, and the system includes a gripper R1 configured to grip a molded product P as a target with use of a jaw R2, and a controller 0 configured to measure properties other than hardness of the molded product P without breaking the molded product P gripped by the gripper R1, and subsequently increase gripping force while the molded product P is kept gripped by the gripper R1 to break the molded product and measure the hardness of the molded product P.

## Description

### Field of the Invention

The invention relates to a system and a method for measurement of hardness and properties other than the hardness of a target molded product.

### Background of the Invention

There has been known a rotary compression molding machine (or a tableting machine; see JP 2020-049516 A or the like) which includes a die table of a turret having die bores, and an upper punch and a lower punch slidably retained above and below each of the die bores, and which is configured to horizontally rotate the turret and the punches together and compression mold a powdery material filled in the die bores when the paired upper and lower punches pass between an upper roll and a lower roll to obtain a molded product. The molding machine of this type is preferably adopted for production of pharmaceutical tablets, food products, electronic components, and the like.

A production situation needs checking whether there is abnormality by on demand inspection of properties of molded products such as size, weight, composition, and hardness. Quality of the molded products are typically controlled by sampling a plurality of molded products out of mass-produced molded products and measuring properties of the molded products thus sampled. Target molded products are picked up often with use of a robot arm or the like including a gripper (alternatively, a hand or a chuck; see JP 2019-107739 A or the like).

Hardness of a molded product is measured through destructive inspection. In a case where the molded product is a tablet, the target tablet is sandwiched between two pressing plates, a first one of the pressing plates is shifted at constant speed relatively to a second one of the pressing plates, to measure force immediately before the tablet is broken (see JP 2771792 B2, "The tablet hardness measurement method", (online), Pharmaceuticals and Medical Devices Agency, (searched on February 1, 2023), and the like). Note that hardness obtained by this measurement method does not conform to definition of hardness in the field of material science or the like (surface resistance against penetration or push fitting with use of a small probe). Each of the pressing plates in contact with a molded product during measurement of hardness need to be appropriately replaced with a member having an outward shape according to size or shape of the target molded product (in many measurement instruments).

Furthermore, to measure hardness and properties other than the hardness of the molded product, the molded product is to be conveyed between a plurality of places having various types of measurement instruments. Every time the target molded product is conveyed, picking up and placing are executed by gripping and lifting up the molded product with use of a robot arm or the like, shifting the molded product to a next site, and lowering and releasing the molded product. Accordingly, inspection of a plurality of properties of the molded product requires takt time. In addition, a large area is occupied because various measurement instruments need to be placed for measurement of the plurality of properties.

### Summary of the Invention

It is a principal object of the invention to shorten takt time necessary for measurement of hardness and properties other than the hardness of a molded product.

The invention provides a hardness measurement system for a molded product, and the system includes: a gripper configured to grip a molded product as a target with use of a jaw; and a controller configured to measure properties other than hardness of the molded product without breaking the molded product gripped by the gripper, and subsequently increase a gripping force while the molded product is kept gripped by the gripper to break the molded product and measure the hardness of the molded product.

A robot arm or the like configured to grip a molded product with use of a gripper and convey the molded product is usually not configured to measure hardness through destruction of the molded product. In contrast, the system according to the invention is configured to measure properties other than the hardness of the target molded product with use of a robot arm or the like, and switch to destructive inspection to measure the hardness of the molded product that is kept gripped. The invention reduces the number of times of picking up and placing to shorten takt time necessary for measurement of the properties of the molded product.

Typically, the controller measures an external size of the molded product gripped by the gripper, and subsequently breaks the molded product kept gripped by the gripper to measure the hardness of the molded product.

The controller estimates the hardness of the molded product in accordance with magnitude of electric current flowing to a motor configured to drive the jaw when the molded product gripped by the gripper is broken.

The molded product as a target is exemplarily obtained by compression molding a powdery material with use of a compression molding machine. A powdery material is an aggregate of minute solids and conceptually includes an aggregate of particles such as so-called granules and an aggregate of powder smaller than such particles.

The invention further provides a hardness measurement method for a molded product, and the method includes: measuring properties other than hardness of the molded product without breaking the molded product as a target gripped by a gripper configured to grip a molded product with use of a jaw; and subsequently increasing gripping force with the molded product kept gripped by the gripper to break the molded product and measure the hardness of the molded product.

### Effects of the Invention

The invention shortens takt time necessary for measurement of hardness and properties other than the hardness of a molded product.

### Brief description of the drawings

Fig. 1 is a side sectional view of a rotary compression molding machine according to an embodiment of the invention.
Fig. 2 is a plan view of a turret included in the rotary compression molding machine.
Fig. 3 is a developed view illustrating vertical shift of punches along with rotation of the turret in the rotary compression molding machine.
Fig. 4 is a plan view of a molded product conveying device and a robot arm according to the embodiment.
Fig. 5 is a perspective view of the robot arm including a gripper according to the embodiment and a molded product gripped by the robot arm.
Fig. 6 is a plan view of jaws of the gripper in the robot arm, and a molded product.
Fig. 7 is a hardware resources configuration diagram of a hardness measurement system according to the embodiment.
Fig. 8 is a flowchart showing an exemplary procedure for measurement of specifications of a molded product with use of the hardness measurement system.
Fig. 9 is a graph exemplarily indicating fluctuation of electric current flowing in a motor of the gripper in the robot arm according to the embodiment.
Fig. 10 is a graph indicating a relationship between magnitude of the electric current flowing in the motor of the gripper in the robot arm and magnitude of force for gripping a molded product.

### Description of the preferred embodiment

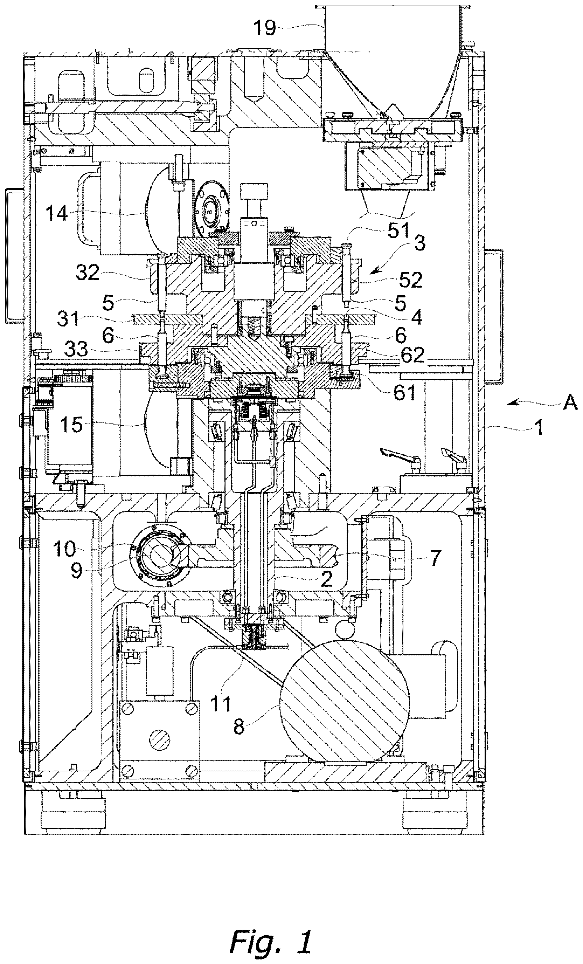

An embodiment of the invention will now be described with reference to the drawings. Initially described is an outline of a rotary compression molding machine A configured to compress a powdery material to produce a molded product P. As shown in Fig. 1, the molding machine A includes a frame 1 accommodating an upright shaft 2 functioning as a rotary shaft and a turret 3 attached to a connection portion that is disposed at the top of the upright shaft 2.

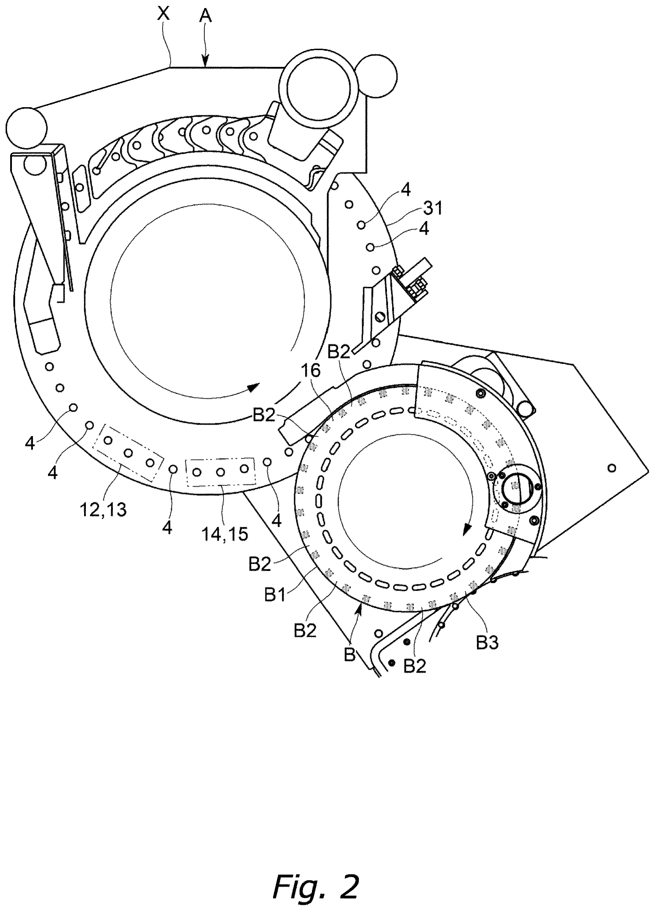

The turret 3 horizontally rotates about the upright shaft 2, more specifically, spins around it. The turret 3 includes a die table (die disc) 31, an upper punch retaining portion 32, and a lower punch retaining portion 33. As shown in Fig. 2, the die table 31 has a substantially circular disc shape and has a plurality of die bores 4 that are disposed in an outer circumferential portion and are aligned in a rotation direction at predetermined intervals. Each of the die bores 4 vertically penetrates the die table 31. The die table 31 may alternatively be divided into a plurality of plates. Instead of the die bores 4 formed by directly drilling the die table 31, the die table 31 may alternatively have a plurality of die members that are separate from the die table 31 and are detachably attached to the die table 31. In this case, each of the die members has a die bore penetrating vertically.

The die bores 4 each have an upper punch 5 and a lower punch 6 disposed above and below the die bore 4. The upper punch 5 and the lower punch 6 are retained by the upper punch retaining portion 32 and the lower punch retaining portion 33 so as to be independently slidable vertically with respect to corresponding one of the die bores 4. The upper punches 5 each have a tip 53 that enters and exits a corresponding one of the die bores 4. The lower punches 6 each have a tip 63 that is kept inserted in corresponding one of the die bores 4. The upper punches 5 and the lower punches 6 horizontally rotate, more specifically revolve, about the upright shaft 2 along with the turret 3 and the die bores 4.

The upright shaft 2 has the lower end to which a worm wheel 7 is attached. The worm wheel 7 meshes with a worm gear 10. The worm gear 10 is fixed to a gear shaft 9 that is driven by a motor 8. Drive power outputted from the motor 8 is transmitted to the gear shaft 9 through a belt 11, so as to drive to rotate the upright shaft 2 by way of the worm gear 10 and the worm wheel 7, and further to rotate the turret 3 and the punches 5 and 6. A rotation angle and rotational speed of each of the turret 3 and the punches 5 and 6 can be detected with use of an angular position sensor such as a rotary encoder.

A powdery material as a raw material for a compression molded product P like a pharmaceutical tablet is fed from a powdery material feeding device (not shown) to a hopper 19, and is fed from the hopper 19 to a feeder X. The hopper 19 is detachably attached to the molding machine A. The powdery material is filled into the die bores 4 from the feeder X. Examples of the feeder X include an agitated feeder and a gravity feeder, either one of which is applicable to the invention.

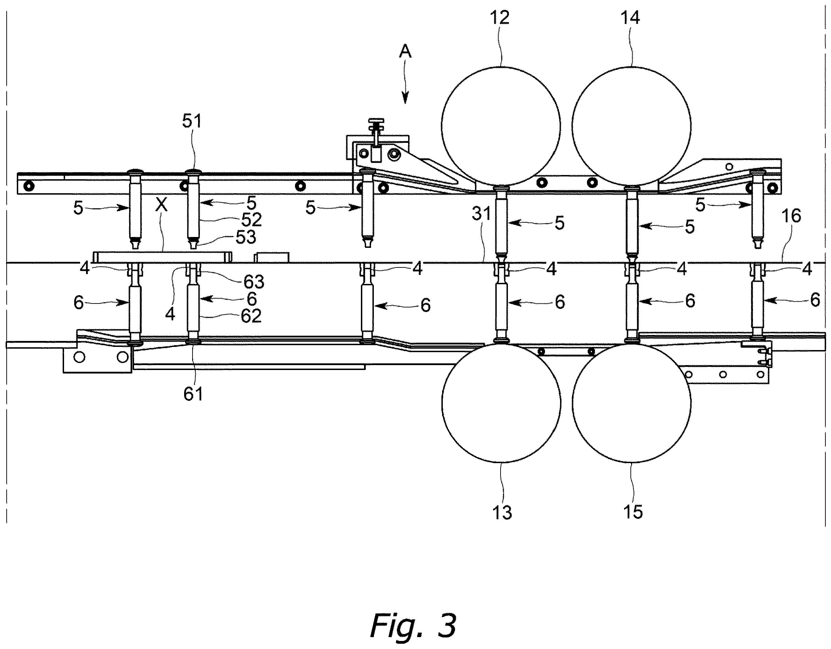

As shown in Figs. 2 and 3, a preliminary compression upper roll 12, a preliminary compression lower roll 13, a substantial compression upper roll 14, and a substantial compression lower roll 15 are disposed on orbits of the punches 5 and 6 that revolve about the upright shaft 2. The preliminary compression upper roll 12 and the preliminary compression lower roll 13 are paired to vertically sandwich the punches 5 and 6, and the substantial compression upper roll 14 and the substantial compression lower roll 15 are paired to vertically sandwich the punches 5 and 6. The preliminary compression upper roll 12 and the preliminary compression lower roll 13 as well as the substantial compression upper roll 14 and the substantial compression lower roll 15 bias the upper and lower punches 5 and 6 to bring the upper and lower punches 5 and 6 closer to each other, so that tip end surfaces of the tips 53 and 63 compress from above and below the powdery material filled in the die bores 4.

The upper and lower punches 5 and 6 have heads 51 and 61 pressed by the rolls 12, 13, 14, and 15, and trunks 52 and 62 smaller in diameter than the heads 51 and 61. The upper punch retaining portion 32 of the turret 3 vertically slidably retains the trunks 52 of the upper punches 5, whereas the lower punch retaining portion 33 vertically slidably retains the trunks 62 of the lower punches 6. The tips 53 and 63 as distal ends of the trunks 52 and 62 are thinner than the other portions and have diameters substantially equal to an inner diameter of the die bores 4 so as to be inserted to the die bores 4. The punches 5 and 6 revolve to cause the rolls 12, 13, 14, and 15 to come closer to the heads 51 and 61 of the punches 5 and 6. The rolls 12, 13, 14, and 15 come into contact with the heads 51 and 61 to climb onto them. The rolls 12, 13, 14, and 15 further press the upper punches 5 downward and press the lower punches 6 upward. While the rolls 12, 13, 14, and 15 are in contact with flat surfaces of the punches 5 and 6, the punches 5 and 6 keep applying constant pressure to the powdery material in the corresponding die bores 4.

The upper rolls 12 and 14 of the molding machine A each have a load cell functioning as a sensor configured to detect pressure applied to compress the powdery material in the die bores 4 by the rolls 12, 13, 14, and 15 via the punches 5 and 6. Reference to output signals of the load cells enables measurement of magnitude of pressure (preliminary compression pressure) applied to compress the powdery material by the preliminarily compression rolls 12 and 13 and magnitude of pressure (substantial compression pressure) applied to compress the powdery material by the substantial compression rolls 14 and 15. The output signals of the load cells each form a pulse signal train having a peak when each of the pairs of punches 5 and 6 compresses the powdery material in a corresponding one of the die bores 4 with maximum pressure. Counting the number of such pulse trains enables obtaining the number of molded products P produced by the molding machine A per unit time.

There is a delivery position 16 for the molded products P, displaced ahead, in a rotation direction of the turret 3 and the punches 5 and 6, from a position pressed by the substantial compression upper roll 14 and the substantial compression lower roll 15. Each of the lower punches 6 ascends until an upper end surface of the tip 63 of the lower punch 6 becomes substantially flush with an upper end of the die bore 4, or an upper surface of the die table 31 before reaching the delivery position 16, and pushes the molded product Pout of the die bore 4. The molded product P pushed out of the die bore 4 is delivered to a module B of a subsequent conveying device at the delivery position 16.

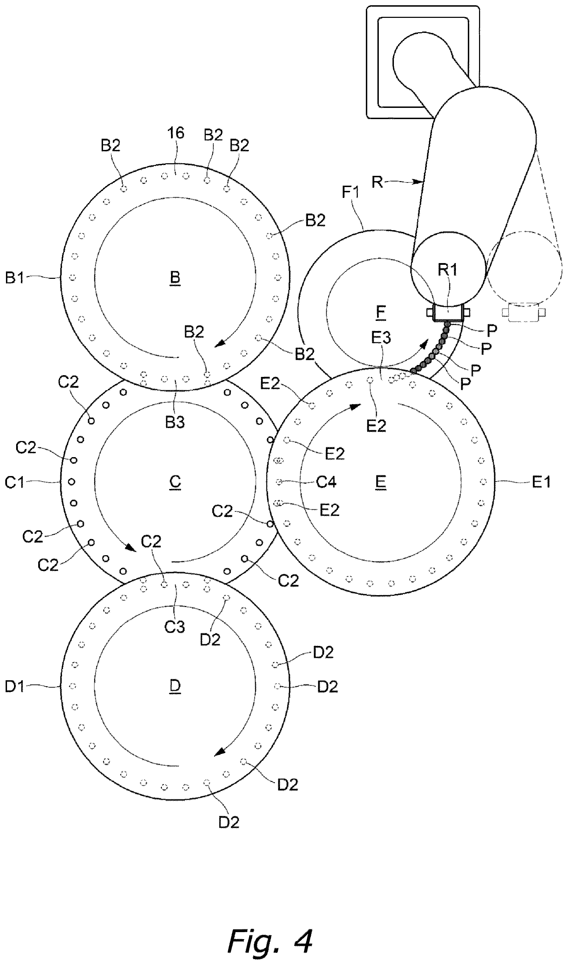

Conveying devices B, C, D, E, and F located downstream of and connected to the molding machine A sequentially convey the molded products P obtained by compression molding the powdery material in the molding machine A. It is possible to simultaneously apply desired post treatment to each of the molded products P being conveyed. Specific examples of such post treatment include removing a powdery material adhering to the surface of the molded product P, printing or engraving on the surface of the molded product P, visual inspection and surface shape inspection of the molded product P, detection of any foreign matter, particularly a metal piece or the like erroneously included in the molded product P, inspection of at least one of properties such as size, weight, density, hardness, and composition of the molded product P. As the peripheral devices B, C, D, E, and F configured to apply post treatment, a powdery material remover, a printer such as an ink jet printer or a laser marking device, an engraving device such as a laser beam machine, a camera sensor, a laser scanner for three-dimensional shape measurement, a metal detector, a device for measurement or inspection of properties of the molded product P, and the like are disposed dispersedly in appropriate modules B, C, D, E, and F. The module D disposed at the most downstream end can have a wrapping device configured to wrap the molded product P in a press through pack (PTP) wrapping sheet, an easy seal open pack (ESOP) wrapping sheet, or the like. As shown in Fig. 4, the embodiment provides the plurality of modules B, C, D, E, and F disposed downstream of the molding machine A, to execute integrally and continuously production of the molded products P by the molding machine A and post treatment by the modules B, C, D, E, and F.

The module B located downstream of and directly connected to the molding machine A is configured to sequentially discharge the molded products P from the molding machine A while the molded products P are kept aligned in the order of molding by the molding machine A. The module B transfers the molded products P with use of a rotator B1 configured to horizontally rotate about a vertical axis in synchronization with the turret 3 and the die table 31 in the molding machine A. The rotator B1 may be driven to rotate by an independent motor separate from the motor 8 of the molding machine A, or the turret 3 of the molding machine A and the rotator B 1 of the module B may be mechanically connected to interlock via a gear transmission mechanism, a winding transmission mechanism, or the like. A rotation angle and rotational speed of the rotator B1 can be detected with use of an angular position sensor such as a rotary encoder.

The rotator B1 has a substantially circular disc shape and has a plurality of capturing portions B2 disposed in an outer circumferential portion and aligned in a rotation direction at predetermined intervals. The capturing portions B2 each receive the single molded product P from the molding machine A at the delivery position 16. That is, each of the capturing portions B2 captures the single molded product P. The capturing portions B2 can thus accommodate the molded products P one by one in the order of alignment of the die bores 4 in the die table 31 of the molding machine A, in other words, while keeping the order of compression molding the molded products P by the molding machine A. Furthermore, the molded products P will not be reversed vertically while being delivered from the die table 31 of the molding machine A to the rotator B1 of the module B. Negative pressure may optionally be applied to the capturing portions B2 of the rotator B1 to pick up the molded products P to the capturing portions B2, respectively, with use of the negative pressure.

The molded products P captured in the capturing portions B2 horizontally rotate along with the rotator B1 to be shifted. The molded products P are delivered to the module C of the subsequent conveying device at a delivery position B3 while keeping the order of alignment.

The module C located downstream of and directly connected to the module B is configured to sequentially discharge the molded products P from the module B while the molded products P are kept aligned in the order of transfer by the module B. The module C transfers the molded products P with use of a rotator C1 configured to horizontally rotate about a vertical axis in synchronization with the rotator B1 of the module B. The rotator C1 may be driven to rotate by an independent motor separate from the motor of the module B, or the rotator B1 of the module B and the rotator C1 of the module C may be mechanically connected to interlock via a gear transmission mechanism, a winding transmission mechanism, or the like. A rotation angle and rotational speed of the rotator C1 can be detected with use of an angular position sensor such as a rotary encoder.

The rotator C1 has a substantially circular disc shape and has a plurality of capturing portions C2 disposed in an outer circumferential portion and aligned in a rotation direction at predetermined intervals. The capturing portions C2 each receive the single molded product P from the module B at the delivery position B3. That is, each of the capturing portions C2 captures the single molded product P. The capturing portions C2 can thus accommodate the molded products P one by one in the order of alignment of the die bores 4 in the die table 31 of the molding machine A, in other words, while keeping the order of compression molding the molded products P by the molding machine A. Furthermore, the molded products P will not be reversed vertically while being delivered from the rotator B1 of the module B to the rotator C1 of the module C. Negative pressure may optionally be applied to the capturing portions C2 of the rotator C 1 to pick up the molded products P to the capturing portions C2, respectively, with use of the negative pressure.

The molded products P captured in the capturing portions C2 horizontally rotate along with the rotator C1 to be shifted. The molded products P are delivered to the module D of the subsequent conveying device at a delivery position C3 while ordinarily keeping the order of alignment. Some of the molded products P may be sampled. Such sampling target molded products P are not delivered to the module D but shift to reach a delivery position C4 beyond the delivery position C3, so as to be delivered to the module E of the conveying device.

The module D located downstream of and directly connected to the module C is configured to sequentially discharge the molded products P from the module C while the molded products P are kept aligned in the order of transfer by the module C. The module D transfers the molded products P with use of a rotator D1 configured to horizontally rotate about a vertical axis in synchronization with the rotator C1 of the module C. The rotator D1 may be driven to rotate by an independent motor separate from the motor of the module C, or the rotator C 1 of the module C and the rotator D 1 of the module D may be mechanically connected to interlock via a gear transmission mechanism, a winding transmission mechanism, or the like. A rotation angle and rotational speed of the rotator D1 can be detected with use of an angular position sensor such as a rotary encoder.

The rotator D1 has a substantially circular disc shape and has a plurality of capturing portions D2 disposed in an outer circumferential portion and aligned in a rotation direction at predetermined intervals. The capturing portions D2 each receive the single molded product P from the module C at the delivery position C3. That is, each of the capturing portions D2 captures the single molded product P. The capturing portions D2 can thus accommodate the molded products P one by one in the order of alignment of the die bores 4 in the die table 31 of the molding machine A, in other words, while keeping the order of compression molding the molded products P by the molding machine A. Furthermore, the molded products P will not be reversed vertically while being delivered from the rotator C 1 of the module C to the rotator D 1 of the module D. Negative pressure may optionally be applied to the capturing portions D2 of the rotator D 1 to pick up the molded products P to the capturing portions D2, respectively, with use of the negative pressure.

The molded products P captured in the capturing portions D2 horizontally rotate along with the rotator D1 to be shifted. The molded products P are collected as products at a predetermined collecting position.

The module E disposed adjacent to the module C is configured to discharge the sampling target molded products P from the module C and convey while the molded products P are kept aligned in the order of transfer by the module C. The module E transfers the molded products P with use of a rotator E1 configured to horizontally rotate about a vertical axis in synchronization with the rotator C1 of the module C. Rotation of the rotator E1 may be stopped while the molded products P are not sampled, that is, the molded products P do not flow to the delivery position C4 and there is no need to transfer the sampling target molded products P. The rotator E1 may be driven to rotate by an independent motor separate from the motor of the module C, or the rotator C1 of the module C and the rotator E1 of the module E may be mechanically connected to interlock via a gear transmission mechanism, a winding transmission mechanism, or the like. A rotation angle and rotational speed of the rotator E1 can be detected with use of an angular position sensor such as a rotary encoder.

The rotator E1 has a substantially circular disc shape and has a plurality of capturing portions E2 disposed in an outer circumferential portion and aligned in a rotation direction at predetermined intervals. The capturing portions E2 each receive the single molded product P from the module C at the delivery position C4. Each of the capturing portions E2 will not capture two or more molded products P. The capturing portions E2 can thus accommodate the molded products P in the order of alignment of the die bores 4 in the die table 31 of the molding machine A, in other words, while keeping the order of compression molding the molded products P by the molding machine A. Furthermore, the molded products P will not be reversed vertically while being delivered from the rotator C1 of the module C to the rotator E1 of the module E. Negative pressure may optionally be applied to the capturing portions E2 of the rotator E1 to pick up the molded products P to the capturing portions E2, respectively, with use of the negative pressure.

The sampling target molded products P captured in the capturing portions E2 horizontally rotate along with the rotator E1 to be shifted. The molded products P are delivered to the module F of the subsequent conveying device at a delivery position E3 while keeping the order of alignment.

The module F located downstream of and directly connected to the module E is configured to discharge the sampling target molded products P from the module E while the molded products P are kept aligned in the order of transfer by the module E. The module F transfers the molded products P with use of a rotator F1 configured to horizontally rotate about a vertical axis in synchronization with the rotator E1 of the module E. Rotation of the rotator F1 may be stopped while the molded products P are not sampled. The rotator F1 may be driven to rotate by an independent motor separate from the motor of the module E, or the rotator E1 of the module E and the rotator F1 of the module F may be mechanically connected to interlock via a gear transmission mechanism, a winding transmission mechanism, or the like. A rotation angle and rotational speed of the rotator F1 can be detected with use of an angular position sensor such as a rotary encoder.

The rotator F1 has a substantially circular disc shape and receives the sampling target molded products P from the module E at the delivery position E3, and the molded products P are deposited on the upper surface of the rotator F1. The sampling target molded products P shift to a position where a robot arm R is located along a rail or a guide (not shown) extending in a rotation direction. The molded products P can thus be picked up in the order of alignment of the die bores 4 in the die table 31 of the molding machine A, in other words, while keeping the order of compression molding the molded products P by the molding machine A. Furthermore, the molded products P will not be reversed vertically while being delivered from the rotator E1 of the module E to the rotator F1 of the module F.

The modules B, C, D, E, and F of the conveying devices can be configured in accordance with JP 2020-049516 A as Japanese Unexamined Patent Publication, or the like.

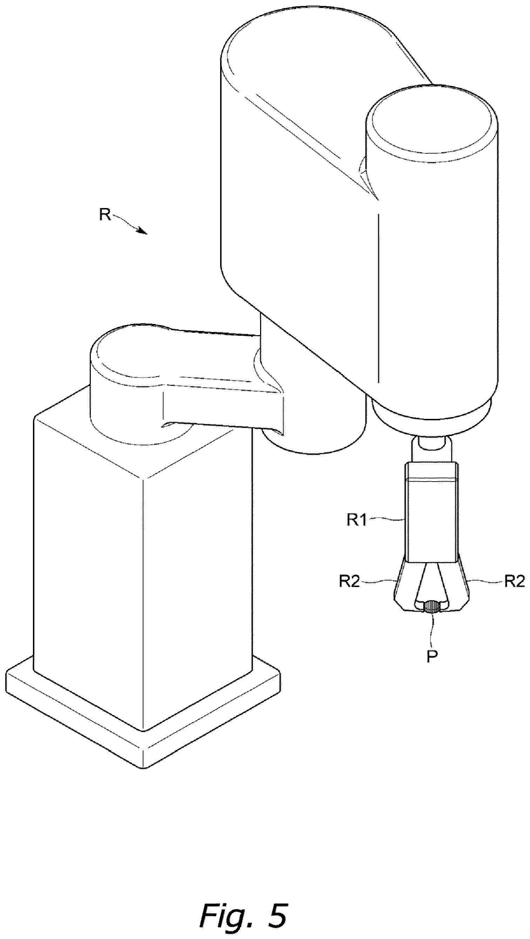

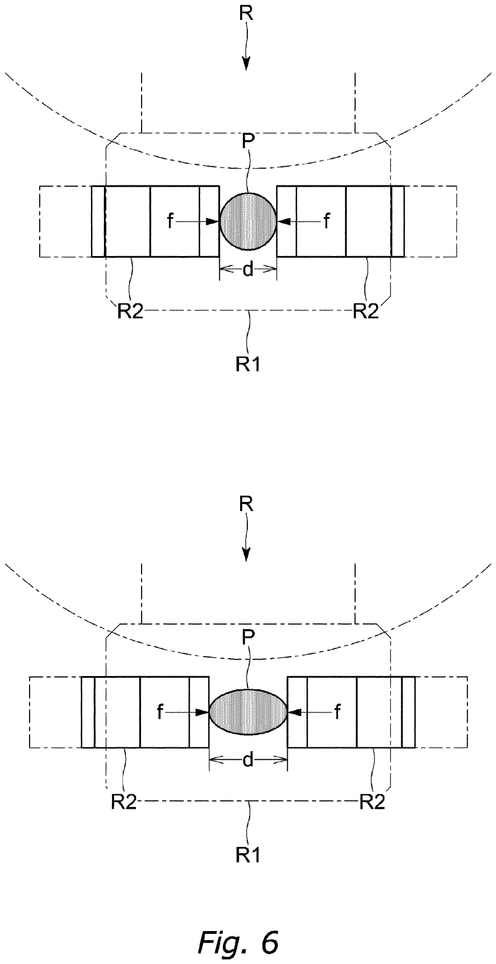

The robot arm R includes a gripper (alternatively, a hand or a chuck) R1, and picks up to convey the sampling target molded products P one by one. As shown in Figs. 5 and 6, the gripper R1 grips the molded product P with use of a plurality of jaws R2. Exemplarily shown is a case where the molded product P is sandwiched between two jaws R2 facing each other. Alternatively, the molded product P may be gripped with use of three or more jaws.

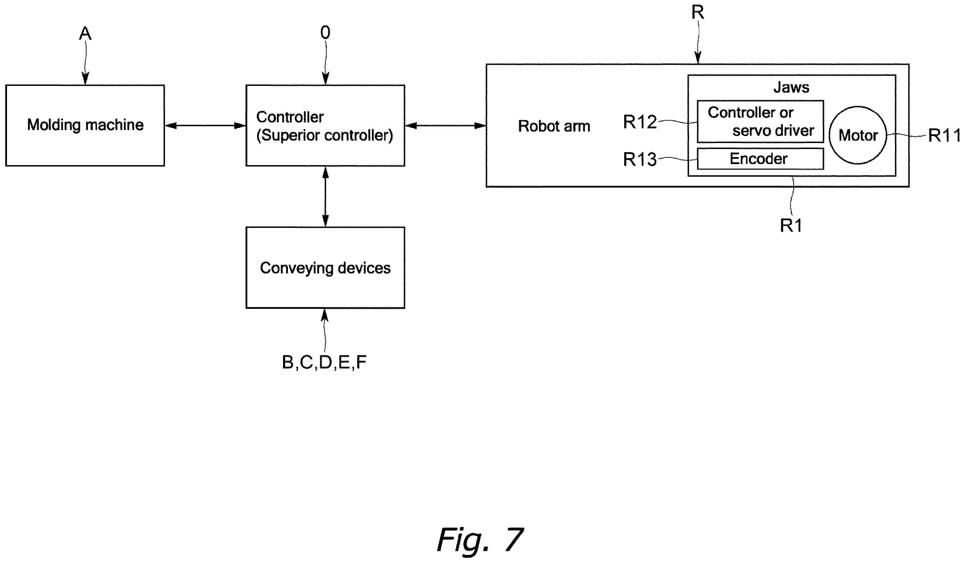

As shown in Fig. 7, the gripper R1 is of a known electric type, and includes a motor R11 configured to drive the jaws R2, a controller or servo driver R12 configured to apply necessary electric current to the motor R11 to control the motor R11 and the jaws R2, and an encoder R13 configured to detect displacement magnitude of the jaws R2. Examples of the motor R11 include a stepping motor and a servo motor. The motor R11 and each of the jaws R2 are mechanically connected via a gear transmission mechanism such as a rack and pinion, or a worm gear. The encoder R13 may be an angular position sensor such as a rotary encoder, which is configured to detect a rotation angle of the motor R11.

The molding machine A, the conveying devices B, C, D, E, and F, the robot arm R (including the gripper R1), a measurement instrument, and the like are controlled by a controller 0 as a superior controller, examples of which include a microcomputer system, a personal computer, and a work station each including a processor, a main memory and an auxiliary storage device (e.g., a flash memory or a hard disk drive), an input/output interface, and the like, as well as a programmable logic controller. The controller 0 is configured to transmit necessary control signals to, as well as receive various signals outputted from, the molding machine A, the conveying devices B, C, D, E, and F, the robot arm R, the measurement instrument, and the like, each of which are connected by wire or wirelessly via the input/output interface. The controller 0 reads a program preliminarily stored in the auxiliary storage device into the processor via the memory, causes the processor to decode the program, and variously controls the molding machine A, the conveying devices B, C, D, E, and F, the robot arm R, and the like.

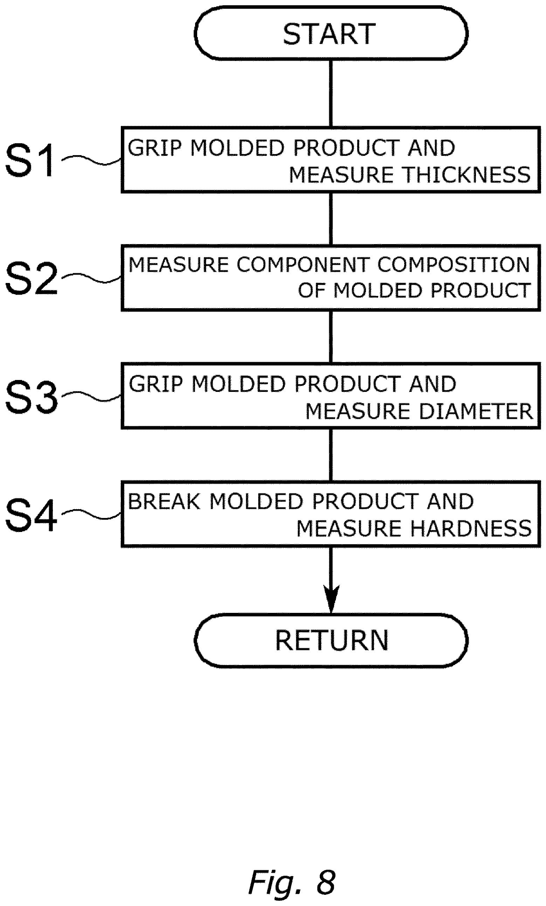

The controller 0 measures various properties (specifications) of the sampling target molded product P picked up by the robot arm R. As shown in Fig. 8, the system according to the embodiment sequentially executes measurement of thickness of the target molded product P (step S 1), measurement of component composition (step S2), measurement of outer diameter (step S3), and measurement of hardness (step S4).

The thickness of the molded product P corresponds to external size in a thickness (vertical) direction perpendicular to a direction of an outer diameter d of the molded product P shown in Fig. 6. The outer diameter d of the molded product P corresponds to a diameter of the molded product P having a perfect circle shape or a substantially perfect circle shape in a planar view as shown exemplarily in an upper portion of Fig. 6, or a longer diameter in a major axis direction of the molded product P having an oval (elliptical, flat oval, or similar) shape in a planar view as shown exemplarily in a lower portion of Fig. 6. Alternatively measured is a shorter diameter in a minor axis direction of the molded product P having the oval shape in a planar view.

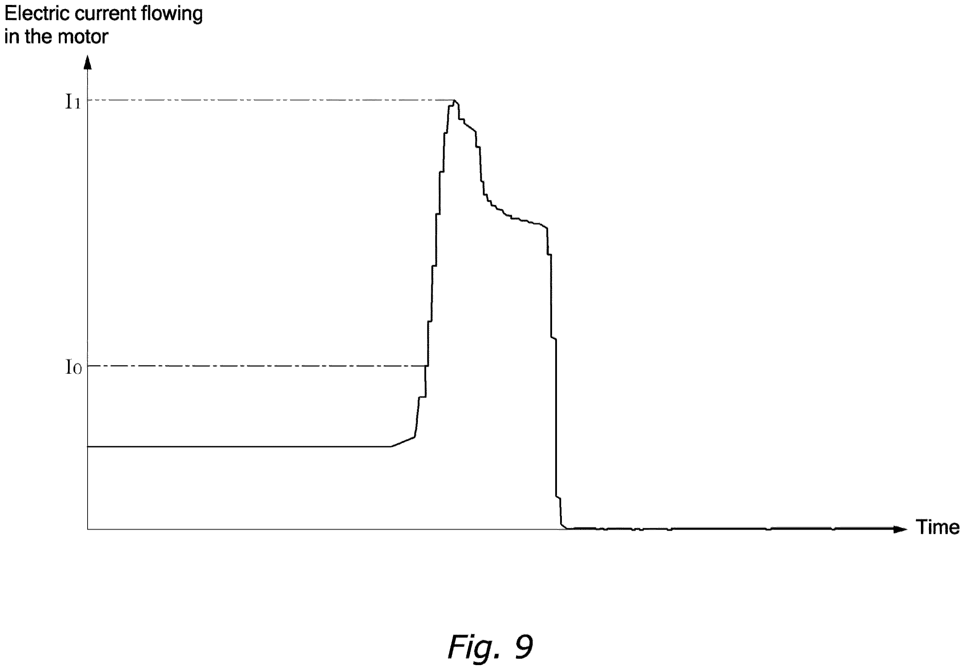

Size measurement in step S 1 and Step S3 can be executed while the molded product P is gripped by the gripper R1 of the robot arm R. When electric current is applied to the motor R11 of the gripper R1, the jaws R2 are displaced to approach the outer surface of the molded product P to eventually come into contact with the outer surface. During this course, electric current flowing in the motor R11 fluctuates as indicated in Fig. 9. When the jaws R2 come into contact with the molded product P and cannot be displaced any more, electric current flowing in the motor R11 increases and force f of the jaws R2 pressing the molded product P increases simultaneously. The controller 0 constantly monitors magnitude of electric current flowing in the motor R11 by referring to an output signal of the controller or servo driver R12. When a value of the electric current increases to exceed a threshold I₀, the controller 0 determines that the jaws R2 perfectly grip the molded product P. The controller 0 refers to an output signal of the encoder R13 to obtain positions of the jaws R2 (distance between the plurality of jaws R2) at the timing, as well as size of the molded product P.

In a case where the robot arm R or the gripper R1 includes a weight detector such as a load cell, weight of the molded product P being gripped can be measured in step S 1 or step S3.

Composition measurement in step S2 is executed with use of a measurement instrument disposed separately from the robot arm R and connected to the controller 0. Examples of the measurement instrument include a camera sensor configured to photograph the molded product P to obtain image data for image analysis, and an analyzer (a Near-InfraRed Spectroscopy analyzer) configured to irradiate the molded product P with electromagnetic waves or light waves (specifically far-red light) to receive and analyze transmitted light or reflected light. In step S2, the robot arm R conveys the target molded product P gripped by the gripper R1 to the measurement instrument, and opens the jaws R2 at the position to release the target molded product P. When the measurement instrument finishes measurement, the robot arm R picks up the molded product P again, in other words, grips and lifts up the molded product P with use of the jaws R2, and conveys the molded product P to outside the measurement instrument. Measurement in step S3 is subsequently executed to the molded product P thus gripped.

Measurement in step S1 to S3 corresponds to nondestructive inspection, whereas hardness measurement in step S4 corresponds to destructive inspection. After the external size d (and weight) of the molded product P gripped by the gripper R1 of the robot arm R is measured in step S3, the procedure switches to hardness measurement in step S4 while the gripper R1 keeps gripping the molded product P without releasing. In step S4, electric current applied to the motor R11 is gradually increased to enhance gripping force f applied to the molded product P already gripped in step S3. Position and direction of application the gripping force f are identical to position and direction of the external size d measured in step S3.

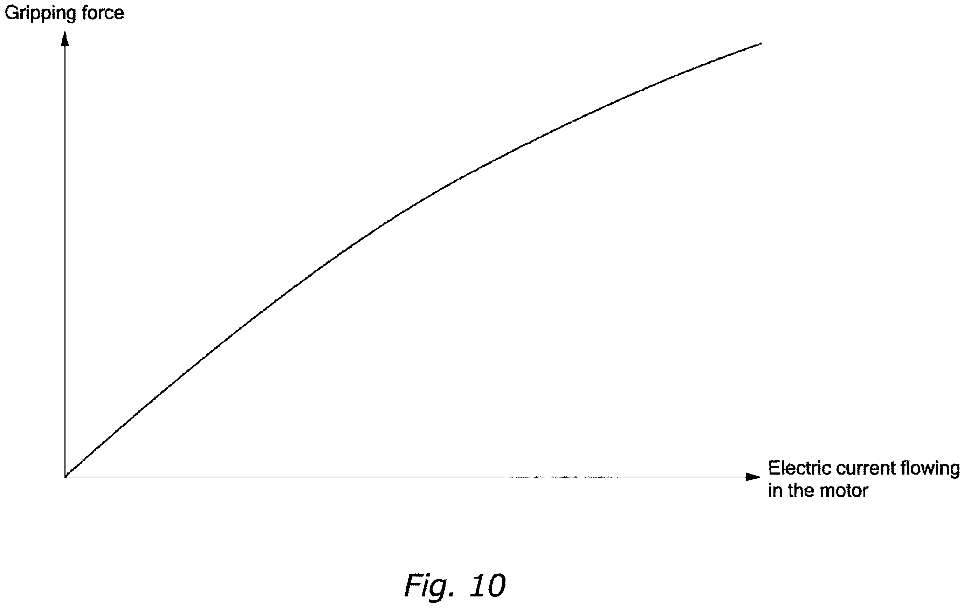

When the gripping force f is extremely strong and the molded product P being gripped is broken, a load applied from the jaws R2 to the motor R11 until immediately before the destruction is rapidly reduced, and accordingly, magnitude of electric current flowing in the motor R11 rapidly decreases. The controller 0 estimates hardness of the target molded product P in accordance with a maximum value I₁ of motor electric current immediately before such rapid decrease. As indicated in Fig. 10, magnitude of electric current applied to the motor R11 and magnitude of the force f applied from the jaws R2 to the molded product P have an approximately linear relationship. That is, the larger the motor electric current, the larger the gripping force f. The controller 0 preliminarily stores and holds, in a memory, data or a function expression, a calibration curve as indicated in Fig. 10, and convers the maximum value I₁ of the motor electric current upon destruction of the molded product P to the force f breaking the molded product P, in other words, hardness of the molded product P.

The embodiment provides the hardness measurement system for the molded product P, the system including: the gripper R1 configured to grip the molded product P as a target with use of the jaws R2; and the controller 0 configured to measure properties (the external size d or weight) other than hardness of the molded product P without breaking the molded product P gripped by the gripper R1, and subsequently increase the gripping force f while the molded product P is kept gripped by the gripper R1 to break the molded product P and measure the hardness of the molded product P.

The embodiment reduces the number of times of picking up and placing upon measurement of hardness and properties other than the hardness of the molded product P, to shorten takt time necessary for measurement. The plurality of properties of the molded product P are measured with use of the single instrument, to reduce an area occupied by the measurement instrument.

Furthermore, there is no need to prepare pressing plates according to size and shape of the molded product P as in conventional hardness measurement. The embodiment thus eliminates time and effort for replacement of pressing plates, and enables measurement of various molded products P.

The invention is not limited to the embodiment detailed above. The gripper R1 is not necessarily included in the robot arm R, and may alternatively be included in a crane configured to be driven in X, Y, and Z axis directions (adopted to a so-called crane game machine or the like), or may be independently configured to measure hardness of the molded product P.

The rotator F1 of the module F for sampling may be replaced with a conveyor configured to linearly convey the molded product P. There may be provided a stationary plate that neither rotates nor linearly shifts. Sampled molded products P may be aligned on the plate (so as to be sequentially pushed out along a rail, guide, or the like (not shown)), to stand by until the molded products P are each gripped by the gripper R1.

The encoder R13 configured to detect displacement magnitude of the jaws R2 may be a potentiometer.

Moreover, specific configurations of the respective portions and the procedure of executed processing can be modified in various manners without departing from the purpose of the invention.

The invention provides a hardness measurement system for a molded product, and the system includes a gripper configured to grip a molded product as a target with use of a jaw, and a controller configured to measure properties other than hardness of the molded product without breaking the molded product gripped by the gripper, and subsequently increase gripping force while the molded product is kept gripped by the gripper to break the molded product and measure the hardness of the molded product.

## Claims

1. A hardness measurement system for a molded product (P), the system comprising:
a gripper (R1) configured to grip a molded product (P) as a target with use of a jaw (R2); and
a controller (0) configured to measure properties other than hardness of the molded product (P) without breaking the molded product (P) gripped by the gripper (R1), and subsequently increase a gripping force while the molded product (P) is kept gripped by the gripper (R1) to break the molded product (P) and measure the hardness of the molded product (P).

2. The hardness measurement system according to claim 1, wherein the controller (0) is configured to measure an external size of the molded product (P) gripped by the gripper (R1), and to subsequently break the molded product (P) kept gripped by the gripper (R1) to measure the hardness of the molded product (P).

3. The hardness measurement system according to claim 1 or 2, wherein the controller (0) is configured to estimate the hardness of the molded product (P) in accordance with magnitude of electric current flowing to a motor (R11) configured to drive the jaw (R2) when the molded product (P) gripped by the gripper (R1) is broken.

4. The hardness measurement system according to any one of claims 1 to 3, wherein the molded product (P) as a target is obtained by compression molding a powdery material with use of a compression molding machine (A).

5. A hardness measurement method for a molded product (P), the method comprising:
measuring properties other than hardness of the molded product (P) without breaking the molded product (P) as a target gripped by a gripper (R1) configured to grip the molded product (P) with use of a jaw (R2); and
subsequently applying a force to the molded product (P) kept gripped by the gripper (R1) to break the molded product and measure the hardness of the molded product (P).
